# EUROPÄISCHE PATENTANMELDUNG

(11) **EP 0 673 654 A1**
(43) Veröffentlichungstag der Anmeldung: **27.09.1995**
(21) Anmeldenummer: 95890056.5
(22) Anmeldetag: 17.03.1995
(51) Int. Cl.: A61K 35/78

(54) **Symphytum-Pflanzen-Extrakt und Verfahren zu seiner Herstellung**

(30) Priorität: 21.03.1994 AT 586/94
(71) Anmelder: Schnecker, Jutta, Dipl.-Ing. Dr., A-1170 Wien (AT)
(72) Erfinder: Schnecker, Jutta, Dipl.-Ing. Dr., A-1170 Wien (AT)

(57) **Zusammenfassung**

Symphytum-Extrakt mit einem Pyrrolizidinalkaloidgehalt von höchstens 0,001 mg/ml Extrakt bei einem Verhältnis Pflanzenteile : Extraktionsmittel von 0,7 : 1 bis 1,7 : 1. Verfahren zur Herstellung eines Symphytum-Extraktes, bei dem der Pyrrolizidinalkaloidgehalt auf einen gewünschten Wert reduziert wird. Der Pflanzenschleim enthaltende Extrakt kann in seiner dickflüssigen Konsistenz entweder unmittelbar als Darreichungsform eingesetzt werden oder zu weiteren Arzneispezialitäten verarbeitet werden.

## Beschreibung

Zur Herstellung pflanzlicher Extrakte für medizinische Anwendungen an Menschen oder Tieren werden üblicherweise Methoden aus den in den jeweiligen Ländern gültigen Arzneibüchern herangezogen. Im österreichischen Arzneibuch (1) werden als Extraktionsmethoden die Mazeration und die Perkolation beschrieben. Bei diesen Methoden ist die Behandlung der Pflanzenteile genau vorgegeben, so auch die Dauer der Extraktionszeit:
"Die Mazeration ist eine bei gewöhnlicher Temperatur vorgenommene einmalige Extraktion einer Droge von angegebenem Zerkleinerungsgrad. Die Droge wird mit der vorgeschriebenen Menge des Extraktionsmittels übergossen und in einem gut verschlossenen Gefäß an einem dem Sonnenlicht nicht unmittelbar ausgesetztem Ort unter öfterem Umschütteln bei Zimmertemperatur 6 Tage lang stehen gelassen. Hierauf wird koliert und der Rückstand ausgepreßt; Kolatur und Preßflüssigkeit sind zu vereinigen. Dann läßt man 3 Tage lang an einem kühlen Ort absetzen und filtriert schließlich unter weitgehender Vermeidung eines Verdunstungsverlustes."(Zitat) "Die Perkolation ist eine fortlaufende Extraktion einer Droge von angegebenem Zerkleinerungsgrad bei gewöhnlicher Temperatur."(Zitat)

Aus Extrakten von Symphytum-Pflanzen werden verschiedene Arzneispezialitäten hergestellt wie zum Beispiel die Kytta-Präparate Kytta-Plasma, Kytta-Salbe oder Kytta-Symphytum-Estrakte (2). Diese Arzneimittel werden unter anderem für die Behandlung von Blutergüssen, Verstauchungen und allen jenen Verletzungen eingesetzt, bei denen die Knochenhaut in Mitleidenschaft gezogen wurde.
Jedoch haben sich die in Symphytum-Pflanzen-Extrakten enthaltenen Pyrrolizidinalkaloide als hepatotoxisch, kanzerogen und mutagen erwiesen (3). Deshalb erfolgte bereits vor einigen Jahren in Österreich eine Limitierung des Gehaltes an Pyrrolizidinalkaloiden in Arzneispezialitäten. Seit August 1994 ist das Inverkehrbringen pyrrolizidinalkaloidhaltiger Zubereitungen aus Symphytum-Pflanzen in Österreich verboten (4). In Deutschland (5) und der Schweiz (6) existieren Obergrenzen für Pyrrolizidinalkaloidgehalte für Symphytum enthaltende Arzneispezialitäten.

Die üblichen Extraktionsmethoden haben sich nicht als geeignet erwiesen, Symphytum-Extrakte bereitzustellen, die hinsichtlich ihres Pyrrolizidinalkaloidgehaltes den Anforderungen bezüglich gesundheitlicher Unbedenklichkeit genügen. Aus diesem Grund gibt es verschiedene Versuche, den Pyrrolizidinalkaloidgehalt zu reduzieren, entweder direkt in den Pflanzen durch züchterische Maßnahmen oder im Pflanzenextrakt, beispielsweise durch eine Behandlung des Pflanzenextraktes mit Ionenaustauschern (6).

Aufgrund der gesundheitlichen Auswirkungen der Pyrrolizidinalkaloide in Extrakten von Symphytum-Pflanzen stellte sich die Aufgabe, einen (konzentrierten) Extrakt aus dieser Pflanzenart mit einem möglichst geringen Pyrrolizidinalkaloidgehalt herzustellen. Die Erfindung löst die gestellte Aufgabe dadurch, daß die Extraktionsbedingungen hinsichtlich Verhältnis von Pflanzenteilen zu Extraktionsmittel, Zusammensetzung des Extraktionsmittels, Luftzufuhr, Lichtzufuhr, Extraktionstemperatur und Extraktionszeit so gewählt werden, daß während der Extraktion der Pflanzenteile die Pyrrolizidinalkaloide abgebaut werden.

Bei dem erfindungsgemäßen Verfahren handelt es sich um eine Extraktion mit einer Alkohol-Wasser-Mischung. Zur Herstellung der Alkohol-Wasser-Mischung wird ein Alkoholmit einem Kohlenstoffanteil von C2 bis C3, wie Ethanol oder Isopropanol, eingesetzt. Der Alkohol wird mit einer geeigneten Menge Wasser versetzt, damit ein verdünnter Alkohol im Bereich von 55 bis 95 Prozent Alkoholgehalt erhalten wird. Die zu extrahierenden Pflanzenteile werden in ein Gefäß eingewogen und mit der Alkohol-Wasser-Mischung übergossen. Das Gewichts-Volums-Verhältnis, in dem die Pflanzenteile und der verdünnte Alkohol gemischt werden, liegt vorzugsweise im Bereich von 0,7 : 1 bis 1,7 : 1. Das Gefäß wird luftdicht verschlossen und unter Lichtschutz gelagert. Die Abnahme der Pyrrolizidinalkaloide wird während der Lagerzeit analytisch verfolgt und die Extraktion bis zum Erreichen des gewünschten Pyrrolizidinalkaloidgehaltes im Extrakt weitergeführt. Für Probenahmen kann das Gefäß kurzzeitig geöffnet werden, ohne daß die Extraktion wesentlich beeinträchtigt wird. Durch Luftzutritt zum gebildeten Extrakt wird allerdings seine Farbe im Laufe der weiteren Lagerung dunkler. Zeitweises Umschwenken des geschlossenen Gefäßes zur Durchmischung des gebildeten Extraktes beschleunigt die Abnahme des Pyrrolizidinalkaloidgehaltes.
Durch die beschriebene Vorgangsweise bei der Extraktion von Symphytum-Pflanzen konnte der Pyrrolizidinalkaloidgehalt auf etwa 1/50 der Ausgangskonzentration im Extrakt reduziert werden. Als Ausgangskonzentration ist die maximal gemessene Pyrrolizidinalkaloidkonzentration ab Beginn der Extraktion definiert, da bei diesem Verfahren zuerst die maximal extrahierbare Menge Pyrrolizidinalkaloide extrahiert wird, die dann während der weiteren Extraktion abgebaut wird. So wurde in einem Extrakt, der aus einem Ansatz von Pflanzenteilen : Extraktionsmittel im Verhältnis von 1,2 : 1 erhalten worden war, als Ausgangskonzentration ein Pyrrolizidinalkaloidgehalt von 0,05 mg/ml Extrakt gemessen, der durch die weitere Extraktion auf 0,001 mg/ml Extrakt reduziert wurde.

Werden für die Extraktion Wurzeln herangezogen, die zu einem Zeitpunkt geerntet wurden, zu dem keine oberirdischen grünen Pflanzenteile von Symphytum-Pflanzen existieren (zum Beispiel im Winter), dann enthält der fertige Extrakt außerdem noch Allantoinkristalle, die sowohl an den Wurzelteilen als auch an der Gefäßwand abgeschieden wurden.

Der fertige Extrakt wird von den Wurzeln abgetrennt. Er kann durch schonendes Rotationsverdampfen eingeengt werden. Er ist sowohl in der ursprünglichen Zusammensetzung als auch in der eingeengten Form über mehrere Jahre haltbar. Dünnschichtchromatographische Analysen nach der im HAB III (7) angegebenen Methode zeigten ein stabiles Verteilungsmuster der im Extrakt enthaltenen, unter UV-Licht-Bestrahlung bei 366 nm und durch Fluoreszenzlöschung bei 254 nm detektierbaren Substanzen. Für die dünnschichtchromatographische Analyse wurden Kieselgel 60-HPTLC-Fertigplatten mit Fluoreszenzindikator für 254 nm gewählt. Das Laufmittel bestand aus Butanol-Eisessig-Wasser im Verhältnis 4 : 1 : 1. Nach der Entwicklung der Platte wurden die einzelnen Substanzbanden durch Fluoreszenzlöschung bei 254 nm und durch Fluoreszenz bei 366 nm detektiert.

Der von den Wurzeln abgetrennte, Pflanzenschleim enthaltende Extrakt kann aufgrund seiner dickflüssigen Konsistenz direkt für medizinische Behandlungen an Menschen eingesetzt werden oder zu anderen pharmazeutischen Darreichungsformen weiterverarbeitet werden. So kann dieser Extrakt in verschiedene Salbengrundlagen, die entweder aus natürlichen oder künstlichen Fetten bestehen oder Emulsionen vom Typ "Wasser in Öl" (W/O-Typ) oder vom Typ "Öl in Wasser" (O/W-Typ) sein können oder auch Polyethylenglycolgele oder Hydrogele darstellen, eingearbeitet werden (siehe dazu: Lehrbuch der pharmazeutischen Technologie (8)). Aber auch zu flüssigen Zubereitungen wie Lösungen und Emulsionen kann dieser Extrakt weiterverarbeitet werden. Weitere Angaben zu den unterschiedlichen Arzneimittelzubereitungen sind in den in den jeweiligen Ländern gültigen Arzneibüchern zu finden.

Anhand der folgenden Beispiele wird das Verfahren erläutert, ohne die Erfindung auf diese Beispiele einzuschränken.

### Beispiel 1

Die Wurzeln wurden zu einem Zeitpunkt ausgegraben, als die oberirdischen Pflanzenteile bereits vertrocknet waren. Die Hauptmenge der Wurzeln bestand aus ein bis drei Jahre alten Wurzeln. Sie wurden von der ihnen anhaftenden Erde grob gereinigt und anschließend kurz mit kaltem Wasser gewaschen, um die restliche Erde zu entfernen. Anschließend wurden die Wurzeln in ein bis drei Millimeter dicke Stücke geschnitten. 620 g geschnittene Wurzeln wurden in ein Glasgefäß eingewogen und 475 ml 80 %iger Ethanol zugefügt. Das Gefäß hatte ein Gesamtvolumen von 1200 ml, davon waren 1150 ml mit der Wurzel-Alkohol-Mischung gefüllt. Der Luftraum darüber betrug daher 50 ml. Das Glasgefäß wurde luftdicht verschlossen und bei Raumtemperatur in einem Raum ohne direkte Sonnenbestrahlung gelagert. Während der Extraktion wurde das Gefäß etwa einmal pro Woche zur Durchmischung des gebildeten Extraktes "auf den Kopf gestellt". Nach einigen Wochen zeigten sich die ersten Allantoinkristalle. Für den Nachweis auf Allantoin wurden einige dieser Kristalle in Wasser gelöst. Nach Zusatz von einer einprozentigen p-Dimethylaminobenzaldehydlösung in n-Salzsäure und leichtem Erwärmen am Bunsenbrenner färbte sich die Lösung gelb, was als Nachweis für Allantoin gilt (7). Die Menge an Allantoinkristallen nahm im Laufe der Extraktionszeit zu.
Nach fünf Monaten wurde ein Pyrrolizidinalkaloidgehalt von 0,001 mg/ml Extrakt (der aus dem Wurzel:Extraktionsmittel-Verhältnis von 1,3 : 1 erhalten worden war) auf folgende Weise gemessen:
2 ml Extrakt wurden mit 5 ml Ethanol versetzt und die ausgefallenen Pflanzenschleimstoffe abfiltriert. Zur Reduktion der N-Oxide in freie Alkaloide wurde das Filtrat über eine Chromatographiesäule mit einem Adsorbat von Indigodisulfonat auf einem hochporösen schwachen Anionentauscher als Sauerstoffadsorber eluiert. Das Eluat wurde mit Ammoniak alkalisiert und mit Dichlormethan ausgeschüttelt. Die Dichlormethan-Phase wurde eingedampft, in Methanol aufgenommen und ein Aliquot dieser methanolischen Alkaloidfraktion auf eine HPTLC-Fertigplatte Kieselgel 60 F-254 (20x10 cm) aufgetragen und dünnschichtchromatographisch getrennt.
Das Laufmittel bestand aus 85 ml Dichlormethan, 14 ml Methanol und 1,5 ml Ammoniak 25 %.
Die Entwicklung der Platte erfolgte durch Tauchen in die nachfolgend angegebenen Tauchlösungen:
- Tauchlösung 1:: 0,1 % o-Chloranil in Toluol.
Die Platte wurde nun 60 Sekunden auf 100°C aufgeheizt und anschließend mit Tauchlösung 2 behandelt.
- Tauchlösung 2:: 2 g 4-Dimethylamino-Benzaldehyd,
85 ml Eisessig,
15 ml Salzsäure 32 %.
Die Pyrrolizidinalkaloide erschienen als violette Flecken auf hellem Hintergrund und konnten mit einem Dünnschichtscanner anhand des ebenfalls aufgetragenen Alkaloids Senecionin als Vergleichsalkaloid quantitativ ausgewertet werden.

### Beispiel 2

Die Wurzeln wurden zu einem Zeitpunkt ausgegraben, als die grünen Pflanzenteile bereits etwa 40 cm hoch waren, aber noch nicht blühten. Hauptsächlich wurden ein- bis dreijährige Wurzeln verwendet. Sie wurden mechanisch grob gesäubert und anschließend kurz mit kaltem Wasser gewaschen. Die Wurzeln wurden in ein bis drei Millimeter dicke Stücke geschnitten.
490 g geschnittene Wurzeln wurden in ein Glasgefäß eingewogen und 390 ml 80 %iger Ethanol zugefügt. Das Gesamtvolumen des Glasgefässes betrug 910 ml, davon waren 860 ml mit der Wurzel-Alkohol-Mischung gefüllt. Somit betrug das Volumen des darüberliegenden Luftraumes 50 ml. Das Gefäß wurde luftdicht verschlossen und bei Raumtemperatur in einem durch das Tageslicht nur schwach erhellten Raum gelagert. Während der Extraktionszeit wurde etwa einmal pro Woche das Gefäß umgeschwenkt, um den entstandenen Extrakt zu mischen.
Nach einem Jahr ergab die Analyse mittels Dünnschichtchromatographie (wie unter Beispiel 1 beschrieben) einen Gehalt an Pyrrolizidinalkaloiden unterhalb der Nachweisgrenze, das heißt, der Gehalt der Pyrrolizidinalkaloide war kleiner als 0,0003 mg/ml Extrakt, der aus einem Verhältnis von Wurzel : Extraktionsmittel von 1,25 : 1 erhalten worden war.

### Beispiel 3

Die Wurzeln wurden zu einem Zeitpunkt ausgegraben, als die oberirdischen Pflanzenteile ca. 60 cm hoch waren und die Blütenstände bereits Samen gebildet hatten, die jedoch noch nicht ausgereift waren. Hauptsächlich wurden ein bis drei Jahre alte Wurzeln verwendet. Sie wurden zuerst mechanisch gereinigt und anschließend kurz mit kaltem Wasser gewaschen. Die Wurzeln wurden in ein bis drei Millimeter große Stücke geschnitten.
350 g geschnittene Wurzeln wurden in ein Glasgefäß eingewogen und 311 ml 80 %iger Isopropanol zugefügt. Das Gesamtvolumen des Gefäßes betrug 720 ml. Davon waren 650 ml mit der Wurzel-Alkohol-Mischung gefüllt und 70 ml betrug somit das Luftvolumen darüber. Das Gefäß wurde luftdicht verschlossen und bei Raumtemperatur in einem durch Tageslicht nur schwach erhellten Raum gelagert. Während der Extraktionszeit wurde etwa einmal pro Woche das Gefäß umgeschwenkt, um den entstandenen Extrakt zu mischen.

### Beispiel 4

Die Wurzeln wurden zu einem Zeitpunkt geerntet, als die oberirdischen Pflanzenteile bereits vertrocknet waren. Hauptsächlich wurden ein- bis dreijährige Wurzeln verwendet. Sie wurden grob von der anhaftenden Erde befreit und dann kurz mit kaltem Wasser gewaschen. Die Wurzeln wurden in ein bis drei Millimeter dicke Stücke geschnitten. 650 g geschnittene Wurzeln wurden in ein Glasgefäß eingewogen und 546 ml 80 %iger Ethanol hinzugefügt. Das Gefäß hatte ein Gesamtvolumen von 1200 ml. 1150 ml waren mit der Wurzel-Alkohol-Mischung gefüllt. Der Luftraum darüber betrug daher 50 ml. Das Gefäß wurde luftdicht verschlossen und bei Raumtemperatur in einem durch Tageslicht nur schwach erhellten Raum gelagert. Nach einigen Tagen zeigten sich die ersten Allantoinkristalle, deren Menge im Laufe der Extraktionszeit zunahm.

### Literaturhinweise:

1. Österreichisches Arzneibuch, Ausgabe 1990,
2. Hagers Handbuch der pharmazeutischen Praxis, Springer-Verlag 1958,
3. Teedrogen, Wissenschaftliche Verlagsgesellschaft mbH Stuttgart 1989,
4. Bundesgesetzblatt für die Republik Österreich vom 16.Juli 1993: 469.Verordnung,
5. Pharmazeutische Industrie 54, Nr. 7, 1992
6. R. Jaspersen-Schib, Schweiz. Apotheker-Zeitung 25, S. 755, 128.Jahrgang,
7. HAB III (Vorschläge für das neue Deutsche Homöopathische Arzneibuch), S. 531, 1964,
8. Rudolf Voigt: Lehrbuch der pharmazeutischen Technologie, fünfte, völlig überarbeitete Auflage, 1984.

## Patentansprüche

1. Verfahren zur Herstellung eines Symphytum-Extraktes mit einem verringerten Pyrrolizidinalkaloidgehalt, dadurch gekennzeichnet, daß man Symphytum-Wurzelteile mit einem Extraktionsmittel aus Wasser und einem Alkohol mit einem Kohlenstoffanteil von C2 bis C3 extrahiert, indem man die Wurzelteile in dem Extraktionsmittel ohne weitere externe Luftzufuhr (ausgenommen bei Probenahmen) so lange stehen läßt, bis der Pyrrolizidinalkaloidgehalt den geforderten Wert von höchstens 0,001 mg/ml aufweist. Nach Beendigung der Extraktion werden die Wurzelteile abgetrennt.

2. Verfahren nach Anspruch 1, dadurch gekennzeichnet, daß das Gewichts-/Volumsverhältnis Wurzelteile : Extraktionsmittel von 0,7 : 1 bis 1,7 : 1 beträgt.

3. Verfahren nach Anspruch 1, dadurch gekennzeichnet, daß man als Alkohol Ethanol einsetzt.

4. Verfahren nach einem der Ansprüche 1 bis 3, dadurch gekennzeichnet, daß der Alkoholgehalt des Extraktionsmittels 55 bis 95 Vol.-% beträgt.

5. Verfahren nach einem der Ansprüche 1 bis 4, dadurch gekennzeichnet, daß die Extraktion unter Lichtschutz durchgeführt wird.

6. Verfahren nach einem der Ansprüche 1 bis 5, dadurch gekennzeichnet, daß als Pflanzenteile im Winter geerntete Wurzeln einsetzt werden.

7. Verfahren nach einem der Ansprüche 1 bis 6, dadurch gekennzeichnet, daß man in Abständen den Pyrrolizidinalkaloidgehalt des Extraktes bestimmt : Der Pyrrolizidinalkaloidgehalt wird dünnschichtchromatographisch bestimmt durch Auftragen eines Aliquots Extrakt, der zuvor über eine Chromatographiesäule, enthaltend ein Adsorbat von Indigodisulfonat auf einem hochporösen schwachen Anionentauscher als Sauerstoffadsorber, eluiert, anschließend mit Ammoniak alkalisiert, mit Dichlormethan ausgeschüttelt, eingedampft und in Methanol aufgenommen wurde, auf eine Kieselgel 60 F-254-Dünnschichtchromatographieplatte und chromatographische Auftrennung unter Verwendung des Laufmittels Dichlormethan : Methanol : Ammoniak 25% = 85 : 14 : 1,5. Die Sichtbarmachung der aufgetrennten Pyrrolizidinalkaloide erfolgt durch Tauchen der Dünnschichtchromatographieplatte in eine Lösung bestehend aus 0,1% o-Chloranil in Toluol und anschließend in eine Lösung bestehend aus 2% 4-Dimethylamino-Benzaldehyd in einer Mischung aus 85 ml Eisessig und 15 ml 32% iger Salzsäure. Die als violette Flecken auf hellem Hintergrund sichtbaren Pyrrolizidinalkaloide werden quantitativ durch Scannen und Vergleich der unter den identischen Bedingungen analysierten Vergleichssubstanz Senecionin bestimmt.

8. Extrakt nach Anspruch 1, dadurch gekennzeichnet, daß der Extrakt am Ende der Extraktion einen auf 1/50 verringerten Pyrrolizidinalkaloidgehalt gegenüber der Ausgangskonzentration aufweist.

9. Arzneimittelzubereitung, enthaltend als wirksame Komponente einen Extrakt, hergestellt nach dem Verfahren nach Anspruch 1.
